(19) Europäisches Patentamt
European Patent Office
Office européen des brevets

(11) Veröffentlichungsnummer: **0 205 029**
**A2**

# (12) EUROPÄISCHE PATENTANMELDUNG

(21) Anmeldenummer: **86107154.6**

(22) Anmeldetag: **27.05.86**

(51) Int. Cl.⁴: **C 07 D 215/56**
C 07 D 413/04, C 07 D 471/04
C 07 D 401/04, C 07 D 498/06
C 07 D 471/06, C 07 D 455/04
A 61 K 31/47, A 61 K 31/50
A 61 K 31/535
//(C07D471/04, 221:00, 221:00),
(C07D498/06, 265:00, 221:00),
(C07D471/06, 241:00, 221:00)

(30) Priorität: **07.06.85 DE 3520295**
**13.07.85 DE 3525108**

(43) Veröffentlichungstag der Anmeldung:
**17.12.86 Patentblatt 86/51**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Grohe, Klaus, Dr.**
**Am Wasserturm 10**
**D-5068 Odenthal(DE)**

(72) Erfinder: **Petersen, Uwe, Dr.**
**Auf dem Forst 4**
**D-5090 Leverkusen(DE)**

(72) Erfinder: **Zeiler, Hans-Joachim, Dr.**
**Elsbeeker Strasse 46**
**D-5620 Velbert 15(DE)**

(72) Erfinder: **Metzger, Karl Georg, Dr.**
**Pahlkestrasse 15**
**D-5600 Wuppertal(DE)**

(54) **Antibakteriell wirksame Chinoloncarbonsäureester.**

(57) Die Erfindung betrifft Chinoloncarbonsäureester, Verfahren zu ihrer Herstellung sowie diese Verbindungen enthaltende antibakterielle Mittel für die parenterale Anwendung.

Die Chinoloncarbonsäureester entsprechen den Formeln (I) und (II)

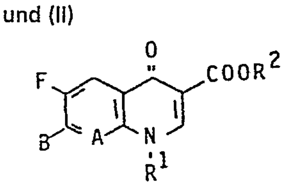

(I)

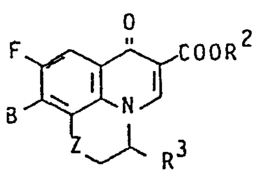

(II)

in denen die Reste A, B, Z, $R_1$, $R_2$ und $R_3$ die in der Beschreibung angegebene Bedeutung haben.

0205029

BAYER AKTIENGESELLSCHAFT
Konzernverwaltung RP
Patentabteilung

5090 Leverkusen, Bayerwerk
Ad/Brü

## Antibakteriell wirksame Chinoloncarbonsäureester

Die Erfindung betrifft Chinoloncarbonsäureester, Verfahren zu ihrer Herstellung sowie diese Verbindungen enthaltende antibakterielle Mittel für die parenterale Anwendung.

Es wurde gefunden, daß die Chinoloncarbonsäureester der Formeln (I) oder (II),

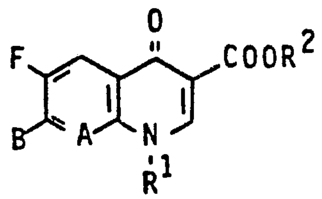

(I)

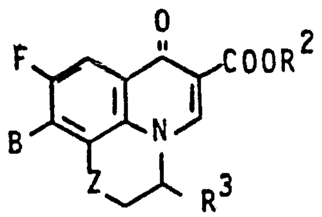

(II)

in denen

A für Stickstoff oder $C-R^4$ steht, wobei

$R^4$ Wasserstoff, Fluor, Chlor oder Methyl bedeutet,

B für 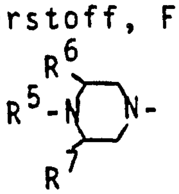 oder 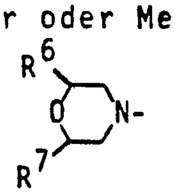 steht und

außerdem B für 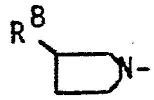- steht, wenn $R^1$ nicht Cyclopropyl bedeutet , worin

$R^5$ Wasserstoff, eine verzweigte oder unverzweigte Alkylgruppe mit 1 bis 4 Kohlenstoffatomen, die gegebenenfalls durch eine Hydroxy- oder Methoxygruppe substituiert sein kann, bedeutet,

$R^6$ für Wasserstoff, Methyl oder Phenyl,

$R^7$ für Wasserstoff oder Methyl und

$R^8$ für Wasserstoff, Hydroxy, Amino, Alkyl- oder Dialkylamino mit 1 oder 2 Kohlenstoffatomen in der Alkylgruppe, Hydroxymethyl, Aminomethyl, Alkyl- oder Dialkylaminomethyl mit 1 oder 2 Kohlenstoffatomen in der Alkylgruppe steht,

$R^1$ einen Alkylrest mit 1 bis 3 Kohlenstoffatomen, Cyclopropyl, 2-Fluorethyl, Methoxy, 4-Fluorphenyl oder Methylamino darstellt,

$R^2$ für gegebenenfalls durch Methoxy oder 2-Methoxyethoxy substituiertes Alkyl mit 1 bis 6 Kohlenstoffatomen sowie Cyclohexyl, Benzyl, 2-Oxopropyl, Phenacyl, Ethoxycarbonylmethyl, Pivaloyloxymethyl steht, sowie

$R^3$ Wasserstoff, Methyl oder Ethyl bedeutet und

Z für Sauerstoff, durch Methyl oder Phenyl substituierten Stickstoff sowie $CH_2$ steht,

und deren pharmazeutisch verwendbaren Hydrate und Säureadditionssalze eine hohe antibakterielle Wirksamkeit aufweisen und sich besonders gut für die parenterale Anwendung eignen. Sie sind im Gegensatz zu den entsprechenden Carbonsäuren sehr gut verträglich und können daher besonders vorteilhaft für parenterale Formulierungen in der Human- und Veterinärmedizin verwendet werden.

Die Erfindung betrifft bevorzugt neue Chinoloncarbonsäureester der Formel (Ia)

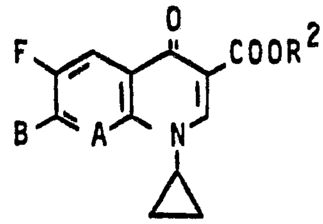

(Ia)

in welcher

B für 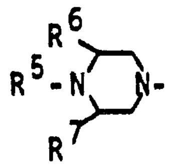 oder 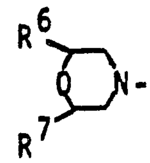 steht und

A, $R^2$, $R^5$, $R^6$ und $R^7$ die oben angegebene Bedeutung haben, sowie deren pharmazeutisch verwendbaren Hydrate und Säureadditionssalze.

Die Erfindung betrifft besonders neue Chinoloncarbonsäureester der Formel (Ia),

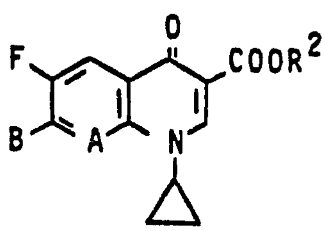

(Ia)

in welcher

$R^2$ für gegebenenfalls durch Methoxy substituiertes Alkyl mit 1 bis 4 Kohlenstoffatomen sowie Benzyl, 2-Oxopropyl, Phenacyl sowie Ethoxycarbonylmethyl steht,

B für 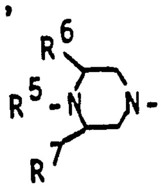 oder 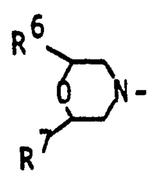 steht, worin

$R^5$ Wasserstoff, Methyl oder Ethyl bedeutet,
$R^6$ für Wasserstoff oder Methyl,
$R^7$ für Wasserstoff oder Methyl steht, und
A die oben angegebene Bedeutung hat,
sowie deren pharmazeutisch verwendbaren Hydrate und Säureadditionssalze.

Weiterhin wurde gefunden, daß man die Verbindungen der Formeln (I) und (II) erhält, wenn man die entsprechenden Chinoloncarbonsäuren (III) bzw. (IV)

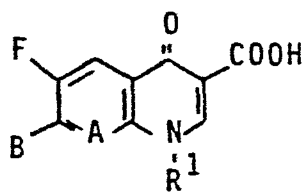

(III)

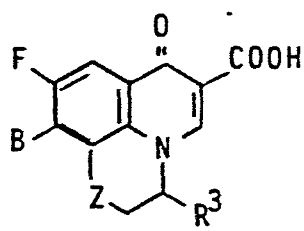

(IV)

in welchen
A, B, $R^1$, $R^3$ und Z die oben angegebene Bedeutung haben, mit Alkoholen der Formel (V)

$$R^2\text{-OH} \quad (V)$$

in welcher
$R^2$ die oben angegebene Bedeutung hat,
in Gegenwart von starken Säuren umsetzt (Methode A).

Die erfindungsgemäßen Verbindungen der Formeln (I) bzw. (II) können auch erhalten werden, indem man Chinoloncarbonsäureester der Formeln (VI) bzw. (VII)

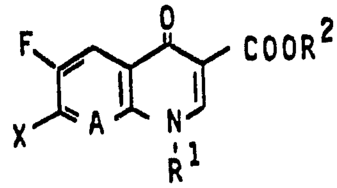

(VI)

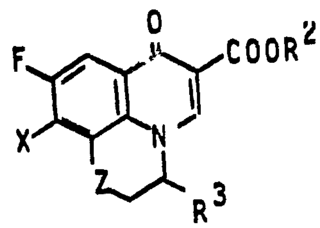

(VII)

in welchen

A, $R^1$, $R^2$, $R^3$ und Z die oben angegebene Bedeutung haben und

X Halogen, bevorzugt Fluor oder Chlor bedeutet,

mit Aminen der Formel (VIII)

B - H (VIII)

in welcher

B die oben angegebene Bedeutung hat,

gegebenenfalls in Gegenwart von Säurebindern umsetzt (Methode B).

Die erfindungsgemäßen Verbindungen der Formeln (I) bzw. (II) können auch erhalten werden, indem man ein Salz einer Carbonsäure der Formel (IX) bzw. (X)

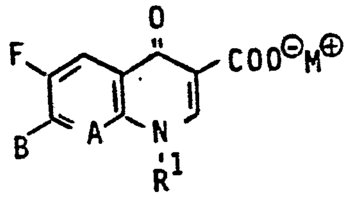

(IX)

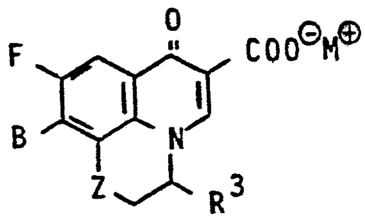

(X)

in welchen
A, B, $R^1$, $R^3$ und Z die ober angegebene Bedeutunq haben

und

$M^\oplus$ für $Na^\oplus$, $K^\oplus$, 1/2 $Ca^{2\oplus}$ oder $Ag^\oplus$ stehen,

mit Verbindungen der Formel (XI),

$$R^2 - Y \qquad (XI)$$

in welcher

$R^2$ die oben angegebene Bedeutung hat und

Y für Halogen, bevorzugt Chlor, Brom oder Jod steht,

umsetzt (Methode C).

Verwendet man nach Methode A 1-Cyclopropyl-7-(4-ethyl-1-piperazinyl)-6-fluor-1,4-dihydro-4-oxo-chinolin-3-carbonsäure und Ethylalkohol als Ausgangsstoffe, so kann der Reaktionsablauf durch das folgende Formelschema wiedergegeben werden:

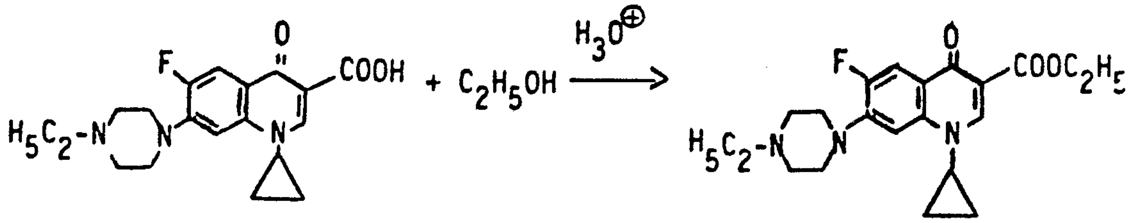


Verwendet man beispielsweise bei der Umsetzung nach Methode B 7-Chlor-1-cyclopropyl-6-fluor-1,4-dihydro-4-oxo-chinolin-3-carbonsäure und 1-Ethylpiperazin als Ausgangsstoffe, so kann der Reaktionsablauf durch das folgende Formelschema wiedergegeben werden:

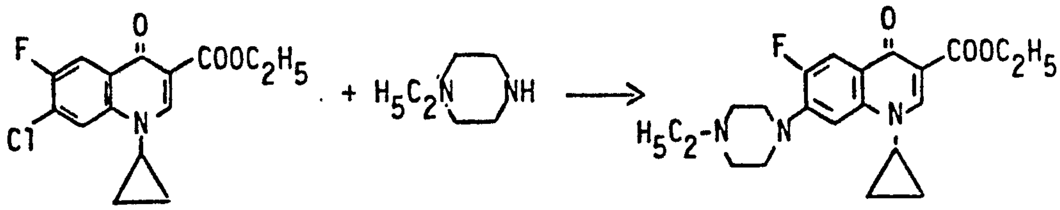

Verwendet man beispielsweise bei der Umsetzung nach Methode C 1-Cyclopropyl-6-fluor-1,4-dihydro-7-(3,5-dimethylmorpholino)-4-oxo-3-chinolincarbonsäure-Natriumsalz und Chloraceton als Ausgangsverbindungen, so kann der Reaktionsablauf durch das folgende Formelschema wiedergegeben werden:

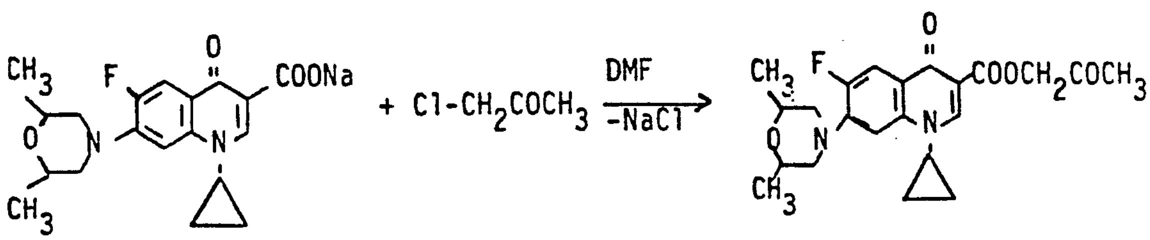


Die als Ausgangsstoffe nach Methode A verwendeten Chinoloncarbonsäuren der Formeln (III) und (IV) sowie die nach Methode B als Ausgangsstoffe verwendeten Chinoloncarbonsäureester (VI) und (VII) sind bekannt oder können nach bekannten Verfahren hergestellt werden.

Als Beispiele seien genannt:
1-Ethyl-6-fluor-1,4-dihydro-4-oxo-7-(1-piperazinyl)-chinolin-3-carbonsäure, 1-Ethyl-6-fluor-1,4-dihydro-7-(4-methyl-1-piperazinyl)-4-oxo-chinolin-3-carbonsäure, 1-Ethyl-6-fluor-1,4-dihydro-4-oxo-7-(1-piperazinyl)-1,8-napthyridin-3-carbonsäure, 9-Fluor-3-methyl-10-(4-methyl-1-piperazinyl)-7-oxo-2,3-dihydro-7,4-pyrido[1,2,3-de]-1,4-benzoxazin-6-carbonsäure, 9-Fluor-5-methyl-8-(4-methyl-1-piperazinyl)-6,7-dihydro-1-oxo-1H,5H-benzo[i,j]chinolicin-2-carbonsäure, 6-Fluor-1-(4-fluorphenyl)-1,4-dihydro-4-oxo-7-(1-piperazinyl)-chinolin-3-carbonsäure, 6-Fluor-1-(2-fluorethyl)-1,4-dihydro-4-oxo-7-(1-piperazinyl)-chinolin-3-carbonsäure, 1-Ethyl-6,8-difluor-1,4-dihydro-4-oxo-7-(4-methyl-1-piperazinyl)-chinolin-3-carbonsäure, 1-Cyclopropyl-6-fluor-1,4-dihydro-4-oxo-7-(1-piperazinyl)-chinolin-3-carbonsäure, 1-Cyclopropyl-6-fluor-1,4-dihydro-4-oxo-7-(4-ethyl-1-piperazinyl)-chinolin-3-carbonsäure, 1-Cyclopropyl-6,8-difluor-1,4-dihydro-4-oxo-7-(1-piperazinyl)-chinolin-3-carbonsäure, 1-Cyclopropyl-6,8-difluor-1,4-dihydro-4-oxo-7-(4-methyl-1-piperazinyl)-chinolin-3-carbonsäure, 1-Cyclopropyl-8-chlor-6-fluor-1,4-dihydro-4-oxo-7-(4-methyl-1-piperazinyl)-chinolin-3-carbonsäure, 1-Cyclopropyl-8-chlor-6-fluor-1,4-dihydro-4-oxo-7-(4-methyl-1-piperazinyl)-chinolin-3-carbonsäure, 1-Cyclopropyl-6-fluor-1,4-dihydro-4-oxo-7-(3,5-dimethylmorphonyl -chinolin-3-carbonsäure, 7-Chlor-1-ethyl-6-fluor-1,4-dihydro-4-oxo-chinolin-3-carbonsäureethylester, 1-Ethyl-6,7-difluor-1,4-dihydro-4-oxo-chinolin-3-carbonsäureethylester, 7-Chlor-1-ethyl-6-fluor-1,4-dihydro-4-oxo-1,8-naphthyridin-3-carbonsäureethylester, 9,10-Difluor-3-methyl-7-oxo-2,3-dihydro-7,4-pyrido[1,2,3-de]-1,4-benzoxacin-6-carbonsäureethylester, 8,9-Difluor-5-methyl-6,7-dihydro-1-oxo-1H,5H-benzo[i,j]chinolicin-2-carbonsäureethylester, 7-Chlor-6-fluor-1-(4-fluorphenyl)-1,4-dihydro-4-oxo-chinolin-3-carbonsäureethylester, 6,7,8-Trifluor-1-(4-fluorphenyl)-1,4-dihydro-4-oxo-chinolin-3-carbonsäureethylester, 7-Chlor-1-cyclopropyl-6-fluor-1,4-dihydro-4-oxo-chinolin-3-carbonsäureethylester, 7-Chlor-1-cyclopropyl-6-fluor-1,4-dihydro-4-oxo-chinolin-3-carbonsäuremethylester, 7-Chlor-1-cyclopropyl-6-fluor-1,4-dihydro-4-oxo-chinolin-3-carbonsäure-n-butylester, 1-Cyclopropyl-6,7-difluor-1,4-dihydro-4-oxo-chinolin-3-carbonsäureethylester, 1-Cyclopropyl-6,7,8-trifluor-1,4-dihydro-4-oxo-chinolin-3-carbonsäureethylester, 8-Chlor-1-cyclopropyl-6,7-difluor-1,4-dihydro-4-oxo-chinolin-3-carbonsäureethylester, 1-Cyclopropyl-7-chlor-6-fluor-1,4-dihydro-4-oxo-1,8-naphthyridin-3-carbonsäureethylester.

Gleichfalls bekannt sind die als Ausgangsstoffe verwendeten Amine der Formel (VIII) (US 4 166 180, J. Med. Chem. 26, 1116 (1983)).

Als Beispiele seien genannt:
Piperazin, N-Methylpiperazin, N-Ethylpiperazin, N-(2-Hydroxyethyl)-piperazin, N-(2-Methoxyethyl)-piperazin, N-Propylpiperazin, N-Isopropylpiperazin, N-Butylpiperazin, N-(sek.-Butyl)-piperazin, 2-Methylpiperazin, cis- und trans-2,6-Dimethylpiperazin, 2-Phenylpiperazin, 2-(4-Fluorphenyl)-piperazin, 2-(4-Chlorphenyl)-piperazin, 2-(4-Methylphenyl)-piperazin, 2-(4-Methoxyphenyl)-piperazin, 2-(4-Hydroxyphenyl)-piperazin, 2-(2-Thienyl)-piperazin, Pyrrolidin, 3-Aminopyrrolidin, 3-Methylamino-pyrrolidin, 3-Aminomethylpyrrolidin, 3-Methylaminomethyl-pyrrolidin, 3-Dimethyl-

0205029

aminomethyl-pyrrolidin, 3-Ethylaminomethyl-pyrrolidin, 3-Hydroxy-pyrrolidin.

Die Umsetzung von (III) bzw. (IV) mit (V) gemäß Methode A wird vorzugsweise in überschüssigem Alkohol (V) als Verdünnungsmittel vorgenommen.

Bei billigen Alkoholen wird hierbei mit einem 5- bis 100-fachen Oberschuß gearbeitet.

Unter starken Säuren versteht man Schwefelsäure, wasserfreien Chlorwasserstoff, Sulfonsäuren wie z.B. Methansulfonsäure oder p-Toluolsulfonsäure und saure Ionenaustauscher.

Die Reaktionstemperaturen liegen zwischen 20 und 200°C, vorzugsweise 60 - 160°C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 15 bar.

Das Reaktionswasser kann auch durch azeotrope Destillation mit Chloroform, Tetrachlorkohlenstoff oder Benzol als Schlepper entfernt werden.

Die Umsetzung von (VI) bzw. (VII) gemäß Methode B mit (VIII) wird vorzugsweise in einem Verdünnungsmittel wie Dimethylsulfoxid, Sulfolan, N-Methylpyrrolidon, N,N-Dimethylformamid, Hexamethyl-phosphorsäuretrisamid, Pyridin, Dioxan oder Tetrahydrofuran vorgenommen.

Als Säurebinder können alle üblichen tertiären organi-

schen Amine und Amidine verwendet werden. Als besonders geeignet seien im einzelnen genannt:

Triethylamin, 1,4-Diaza-bicyclo/2,2,2/-octan (DABCO), 1,8-Diaza-bicyclo/5,4,0/-undec-7-en (DBU) oder überschüssiges Amin (VIII).

Die Reaktionstemperaturen können in einem größeren Bereich variiert werden. Im allgemeinen arbeitet man zwischen etwa 20 und 200°C, vorzugsweise zwischen 80 und 180°C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens setzt man auf 1 Mol des Esters (VI) bzw. (VII) 1 bis 6 Mol, vorzugsweise 1 bis 2 Mol des Amins (VIII) ein.

Die als Ausgangsstoffe nach Methode C verwendeten Salze der Formeln (IX) bzw. (X) sind bekannt oder können nach bekannten Methoden aus der zugrundeliegenden Carbonsäure mit Alkali-, Erdalkali- oder Silberhydroxid erhalten werden.

Die als Ausgangsstoffe verwendeten Halogenverbindungen der Formel (XI) sind bekannt. Als Beispiele seien genannt:
Chloraceton, Bromaceton, Phenacylbromid, Benzylchlorid, Bromessigsäureethylester, Pivaloyloxymethylchlorid, 3-Chlormethyl-2(3H)-benzoxazolon.

Die Umsetzung von (IX) bzw. (X) mit (XI) gemäß Methode C wird vorzugsweise in Dimethylformamid, Dimethylacetamid oder Dimethylsulfoxid als Verdünnungsmittel vorgenommen.

Die Reaktionstemperaturen liegen zwischen etwa 0°C und etwa 150°C, vorzugsweise zwischen 10°C und 50°C.

Die Umsetzung kann bei Normaldruck, aber auch bei erhöhtem Druck durchgeführt werden. Im allgemeinen arbeitet man bei Normaldruck.

Bei der Durchführung des erfindungsgemäßen Verfahrens setzt man auf 1 Mol des Salzes (IX) bzw. (X) 1 bis 5 Mol, vorzugsweise 1 bis 2 Mol der Halogenverbindung (XI) ein.

Die Herstellung der Säureadditionssalze der erfindungsgemäßen Verbindungen erfolgt in üblicher Weise zum Beispiel durch Lösen oder Suspendieren der Ester (I) bzw. (II) in dem entsprechenden Alkohol, Methylenchlorid, Chloroform, Diethylether, Dimethylformamid, Dioxan, Acetonitril oder Tetrahydrofuran und Ausfällen mit mindestens der stöchiometrischen Menge der entsprechenden Säure, eventuell unter Zugabe von Ether. Man kann auch den Ester (I) bzw. (II) in überschüssiger wäßriger Säure lösen und das Salz mit einem mit Wasser mischbaren organischen Lösungsmittel (Methanol, Ethanol, Aceton, Acetonitril) ausfällen. Als pharmazeutisch verwendbare Salze sind beispielsweise die Salze von Salzsäure, Schwefelsäure, Essigsäure, Glykolsäure, Milchsäure, Zitronensäure, Weinsäure, Methansulfonsäure, Galacturonsäure, Gluconsäure, Glutaminsäure, Asparaginsäure oder Embonsäure zu verstehen.

Außer den in den Beispielen aufgeführten Verbindungen seien als Wirkstoffe im einzelnen genannt:

1-Ethyl-6-fluor-1,4-dihydro-4-oxo-7-(1-piperazinyl)-chinolin-3-carbonsäureethylester,
1-Ethyl-6-fluor-1,4-dihydro-4-oxo-7-(1-piperazinyl)-chinolin-3-carbonsäuremethylester,
1-Ethyl-6-fluor-1,4-dihydro-4-oxo-7-(1-piperazinyl)-chinolin-3-carbonsäure-n-butylester,
1-Ethyl-6-fluor-1,4-dihydro-7-(4-methyl-1-piperazinyl)-4-oxo-chinolin-3-carbonsäureethylester,
1-Ethyl-6-fluor-1,4-dihydro-4-oxo-7-(1-piperazinyl)-1,8-naphthyridin-3-carbonsäureethylester,
9-Fluor-3-methyl-10-(4-methyl-1-piperazinyl)-7-oxo-2,3-dihydro-7,4-pyrido/1,2,3-de/1,4-benzoxazin-6-carbonsäureethylester,
9-Fluor-5-methyl-8-(4-methyl-1-piperazinyl)-6,7-dihydro-1-oxo-1H,5H-benzo/i,j/chino lin-2-carbonsäureethylester,
6-Fluor-1-(4-fluorphenyl)-1,4-dihydro-4-oxo-7-(1-piperazinyl)-chinolin-3-carbonsäureethylester,
6-Fluor-1-(2-fluorethyl)-1,4-dihydro-4-oxo-7-(1-piperazinyl)-chinolin-3-carbonsäureethylester,
1-Ethyl-6,8-difluor-1,4-dihydro-4-oxo-7-(1-piperazinyl)-chinolin-3-carbonsäureethylester,
1-Ethyl-6,8-difluor-1,4-dihydro-4-oxo-7-(4-methyl-1-piperazinyl)-chinolin-3-carbonsäure-n-propylester,
1-Cyclopropyl-6,8-difluor-1,4-dihydro-4-oxo-7-(1-piperazinyl)-chinolin-3-carbonsäureethylester,
1-Cyclopropyl-6,8-difluor-1,4-dihydro-4-oxo-7-(4-methyl-1-piperazinyl)-chinolin-3-carbonsäureethylester,
1-Cyclopropyl-6,8-difluor-1,4-dihydro-4-oxo-7-(3-methyl-1-piperazinyl)-chinolin-3-carbonsäure-n-butylester,
1-Cyclopropyl-8-chlor-6-fluor-1,4-dihydro-4-oxo-7-(4-ethyl-1-piperazinyl)-chinolin-3-carbonsäureethylester,
1-Cyclopropyl-6-fluor-1,4-dihydro-4-oxo-8-methyl-7-(4-methyl-1-piperazinyl)-chinolin-3-carbonsäuremethylester und
1-Ethyl-6-fluor-1,4-dihydro-4-oxo-7-(3-amino-1-pyrrolidinyl)-chinolin-3-carbonsäurethylester.

Die erfindungsgemäßen Verbindungen zeigen bei geringer Toxizität ein breites antibakterielles Spektrum gegen gram-positive und gram-negative Keime, insbesondere gegen Enterobakteriaceen; vor allem auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindunsgemäßen Verbindungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Gram-positive Kokken, z. B. Staphylokokken (Staph. aureus, Staph. epidermidis) und Streptokokken (Strept. agalactiae, Strept. faecalis, Strept. pneumoniae, Strept. pyogenes); gram-negative Kokken (Neisseria gonorrheae) sowie gram-negative Stäbchen wie Enterobakteriaceen, z. B. Escherichia coli, Hämophilus influenzae, Citrobacter (Citrob. freundii, Citrob. diversus),Salmonella, Shigella; ferner Klebsiellen (Klebsiella pneumoniae, Klebs. oxytoca), Enterobacter (Ent. aeregenes, Ent. agglomerans), Hafnia, Serratia (Serr. mascescens), Proteus (Pr. mirabilis, Pr. rettgeri, Per. vulgaris) Providencia, Yersinia sowie die Gattung Acinetobacter. Darüberhinaus umfaßt das antibakterielle Spektrum die Gattung Pseudomonas (Ps. aeruginosa, Ps. maltophilia) sowie strikt anaerobe Bakterien wie z. B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z. B. Mycobacterium tuberculosis.

Die obige Aufzählung von Erregern ist lediglich beispielhaft und keineswegs beschränkend aufzufassen. Als Krankheiten, die durch die erfindungsgemäßen Verbindungen verhindert, gebessert und/oder geheilt werden können, seien beispielsweise genannt:

Otitis; Pharyngitis; Pneumonie; Peritonitis; Pyelonephritis; Cystitis; Endocarditis; Systeminfektionen; Bronchitis; Arthritis; lokale Infektionen; septische Erkrankungen.

Zur vorliegenden Erfindung gehören pharmazeutische Zubereitungen zur parenteralen Anwendung, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitung in Form einzelner Teile, z.B. Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind flüssige Verdünnungsmittel, Füllstoffe und Formulierungshilfsmittel jeder Art zu verstehen.

Als bevorzugte pharmazeutische Zubereitungen seien Lösungen, Suspensionen und Emulsionen genannt.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die Wirkstoffe oder die pharmazeutischen Zubereitungen können vorzugsweise parenteral wie intravenös oder intramuskulär appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe, vorzugsweise in Mengen von etwa 1 bis etwa 250, insbesondere 3 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Bevorzugt können die erfindungsgemäßen Verbindungen parenteral auch zur qualitativen und quantitativen Verbesserung tierischer Erzeugnisse verwendet werden.

Die erfindungsgemäßen Verbindungen zeigen eine ausgezeichnete lokale Verträglichkeit, z. B. nach subkutaner Applikation, im Gegensatz zu den freien Carbonsäuren. Dazu wurden Mäuse über 5 Tage 2 x pro Tag mit 1 bis 10 % Lösungen der Wirkstoffe subkutan behandelt, entsprechend einer Dosis von 100 mg/kg bis 300 mg/kg. Während der Behandlungsperiode sowie nach Absetzen der Behandlung wurden die Tiere beobachtet. Es zeigte sich, daß die Mäuse, die mit den erfindungsgemäßen Verbindungen, z. B. Beispiel 1, behandelt worden waren, im Bereich der Injektionsstelle keine Merkmale einer lokalen Gewebeschädigung aufwiesen, während die entsprechenden freien Carbonsäuren zu schwersten nekrotischen Veränderungen und Verhärtungen der Haut und der darunterliegenden Bindegewebsschichten führten. Somit sind die erfindungsgemäßen Verbindungen lokal deutlich besser verträglich als die entsprechenden freien Carbonsäuren und es können Lösungen im oben genannten Konzentrationsbereich bei guter lokaler Verträglichkeit mehrfach angewendet werden. In einem Mäuseprotektionsversuch (Infektion intraperitoneal, Therapie 30 Minuten nach Infektion durch subkutane Gabe) konnte gezeigt werden, daß die Substanzen nach parenteraler Applikation eine gute protektive Wirkung bei erhöhter lokaler Verträglichkeit des Esters besitzen. Die erfindungsgemäßen Verbindungen sind damit in gleicher Dosis protektiv wirksam wie die deutlich schlechter verträglichen freien Carbonsäuren.

Tabelle I

Es wurde im Tierversuch die protektive Wirkung der Substanzen überprüft

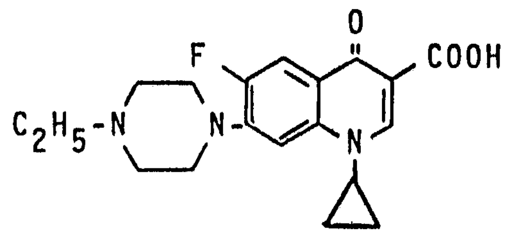

Substanz A

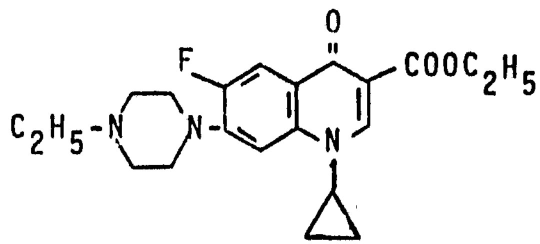

Substanz B

| Substanz | $ED_{80}$ - 100 mg/kg sc |
|---|---|
| A | 2,5 |
| B | 2,5 |

Die folgenden Beispiele erläutern die Erfindung:

Beispiel 1

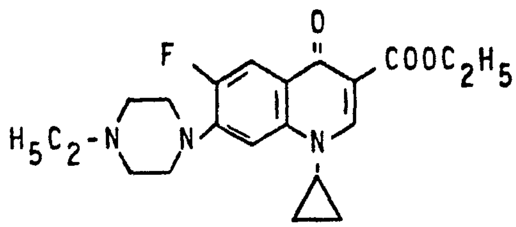

a) Chlorwasserstoff-Verfahren:

In eine Suspension von 184 g 1-Cyclopropyl-7-(4-ethyl-1-piperazinyl)-6-fluor-1,4-dihydro-4-oxo-chinolin-3-carbonsäure in 2750 ml Ethanol wird bei Raumtemperatur beginnend trockener Chlorwasserstoff eingeleitet. Anschließend wird unter weiterem Einleiten von Chlorwasserstoff ca. 14 Stunden refluxiert, die klare Lösung im Vakuum eingeengt, der Rückstand in 2200 ml Eiswasser gelöst und die Lösung mit 800 ml 6 N Natronlauge auf pH = 12-14 gestellt. Der Niederschlag wird rasch abgesaugt, mit Eiswasser gewaschen und im Vakuum bei 100°C getrocknet. Die Umkristallisation aus 750 ml Toluol liefert nach Zugabe von 750 ml Leichtbenzin 140,1 g (70,6 % d.Th.) 1-Cyclopropyl-6-fluor-7-(4-ethyl-1-piperazinyl)-1,4-dihydro-4-oxo-chinolin-3-carbonsäureethylester vom Schmelzpunkt 186-187°C.

b) Schwefelsäure-Verfahren:

Eine Suspension von 18 g 1-Cyclopropyl-7-(4-ethyl-1-piperazinyl)-6-fluor-1,4-dihydro-4-oxo-chinolin-3-carbonsäure in 120 ml Ethanol wird tropfenweise mit 24 g 96%-iger Schwefelsäure versetzt. Man refluxiert 12 Stunden, zieht den überschüssigen Alkohol im Vakuum ab und löst den Rückstand in 200 ml Eiswasser. Unter

Eiskühlung wird mit einer kalten Lösung von 19,2 g Natriumhydroxid in 100 ml Wasser auf pH = 12-14 eingestellt, der gebildete Niederschlag abgesaugt, mit Wasser gewaschen und im Vakuum bei 100°C getrocknet. Nach Umkristallisation aus Toluol/Leichtbenzin werden 10 g (52,5 % d. Th.) 1-Cyclopropyl-7-(4-ethyl-1-piperazinyl)-6-fluor-1,4-dihydro-4-oxo-chinolin-3-carbonsäureethylester vom Schmelzpunkt 187 bis 189 °C erhalten.

c) Umsetzung von 7-Chlor-1-cyclopropyl-6-fluor-1,4-dihydro-4-oxo-chinolin-3-carbonsäureethylester mit 1-Ethylpiperazin

Eine Lösung von 15,5 g 7-Chlor-1-cyclopropyl-6-fluor-1,4-dihydro-4-oxo-chinolin-3-carbonsäureethylester in 60 ml Dimethylsulfoxid wird mit 11,8 g 1-Ethyl-piperazin versetzt und dann 5 Stunden auf 150°C erhitzt. Die heiße Lösung gießt man auf Eis, saugt den Niederschlag ab, wäscht gut mit Wasser nach und trocknet im Vakuum bei 100°C. Nach Umkristallisation aus Toluol/Leichtbenzin in Gegenwart von Tonsil werden 12,2 g 1-Cyclopropyl-7-(4-ethyl-1-piperazinyl)-6-fluor-1,4-dihydro-4-oxo-chinolin-3-carbonsäureethylester vom Schmelzpunkt 185 - 187°C erhalten.

Beispiel 2

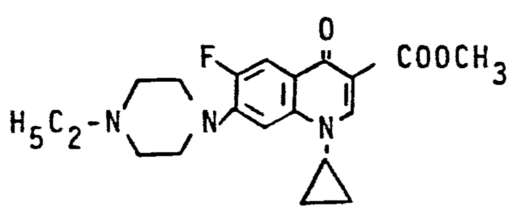

Analog Beispiel 1 (b) wird die Veresterung in Methanol durchgeführt, wobei in 78 %-iger Ausbeute der 1-Cyclopropyl-7-(4-ethyl-1-piperazinyl)-6-fluor-1,4-dihydro-4-oxo-chinolin-3-carbonsäuremethylester vom Schmelzpunkt 218 - 220°C erhalten wird.

Beispiel 3

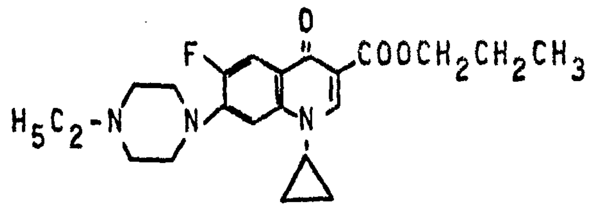

Analog Beispiel 1 (b) wird die Veresterung in n-Propanol durchgeführt, wobei nach 5 Stunden Rückfluß in 58 %-iger Ausbeute der 1-Cyclopropyl-7-(4-ethyl-1-piperazinyl)-6-fluor-1,4-dihydro-4-oxo-chinolin-3-carbonsäure-n-propylester vom Schmelzpunkt 168 - 169°C erhalten wird.

Beispiel 4

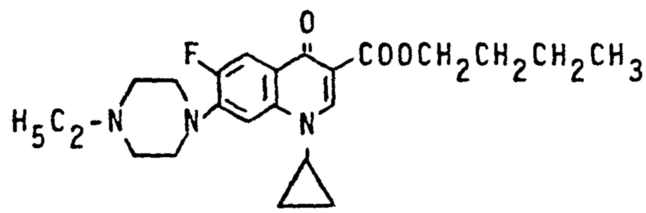

Analog Beispiel 1 (b) wird die Veresterung in n-Butanol durchgeführt, wobei nach 3 Stunden Rückfluß in 56 %-iger Ausbeute der 1-Cyclopropyl-7-(4-ethyl-1-piperazinyl)-6-fluor-1,4-dihydro-4-oxo-chinolin-3-carbonsäure-n-butylester vom Schmelzpunkt 159 - 161°C erhalten wird.

Beispiel 5

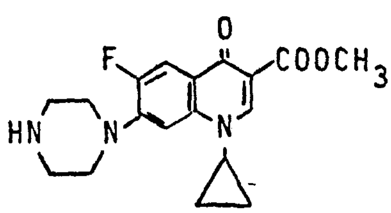

6,6 g (20 mmol) 1-Cyclopropyl-6-fluor-1,4-dihydro-4-oxo-7-(1-piperazinyl)-3-chinolincarbonsäure werden in 150 ml absolutem Methanol suspendiert und bei Rückflußtemperatur wird während 4 Stunden ein trockener HCl-Strom eingeleitet,

wobei sich eine gelbe Lösung bildet. Man engt ein, löst den öligen Rückstand (kristallisiert beim Stehen durch) sogleich in Wasser und stellt mit kalter verdünnter Natronlauge unter Eiskühlung vorsichtig auf pH 8-9. Der ausgefallene Niederschlag wird rasch abgesaugt, mit Eiswasser gewaschen und getrocknet. Man erhält 3,7 g (54 % d.Th.) 1-Cyclopropyl-6-fluor-1,4-dihydro-4-oxo-7-(1-piperazinyl)-3-chinolincarbonsäure-methylester vom Schmelzpunkt 247 - 249°C (nach Umkristallisation aus Methanol: Schmelzpunkt 249 - 251°C).

Beispiel 6

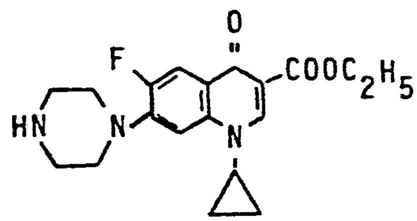

Analog Beispiel 5 wird die Veresterung in Ethanol durchgeführt, wobei 1-Cyclopropyl-6-fluor-1,4-dihydro-4-oxo-7-(1-piperazinyl)-3-chinolincarbonsäureethylester vom Schmelzpunkt 228 - 230°C erhalten wird.

Beispiel 7

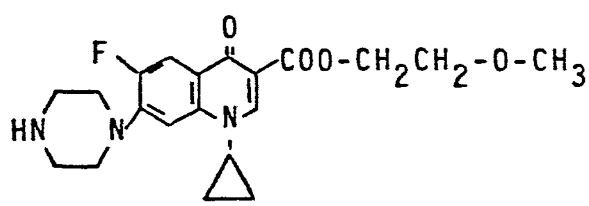

Man arbeitet analog Beispiel 5 in 2-Methoxyethanol und erhält 1-Cyclopropyl-6-fluor-1,4-dihydro-4-oxo-7-(1-piperazinyl)-3-chinolincarbonsäure-(2-methoxyethylester) vom Schmelzpunkt 140 - 142°C.

Beispiel 8

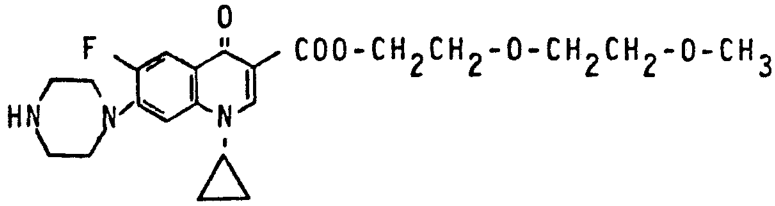

Man arbeitet analog Beispiel 5 in 2-(2-Methoxyethoxy)-ethanol (Diethylenglykolmonomethylether) und erhält 1-Cyclopropyl-6-fluor-1,4-dihydro-4-oxo-7-(1-piperazinyl)-3-chinolincarbonsäure-/2-(2-methoxyethoxy)-ethylester7 vom Schmelzpunkt 182 - 190°C.

Beispiel 9

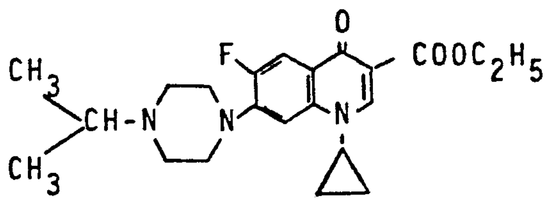

Analog Beispiel 5 wird 1-Cyclopropyl-6-fluor-1,4-dihydro-7-(4-isopropyl-1-piperazinyl)-4-oxo-3-chinolincarbonsäure mit Ethanol verestert. Man erhält 1-Cyclopropyl-6-fluor-1,4-dihydro-7-(4-isopropyl-1-piperazinyl)-4-oxo-3-chinolincarbonsäure-ethylester vom Schmelzpunkt 186 - 187°C.

Die hierfür benötigte 1-Cyclopropyl-6-fluor-1,4-dihydro-7-(4-isopropyl-1-piperazinyl)-4-oxo-3-chinolincarbonsäure wird auf folgendem Wege erhalten:

6,6 g (20 mmol) 1-Cyclopropyl-6-fluor-1,4-dihydro-4-oxo-7-(1-piperazinyl)-3-chinolincarbonsäure werden in 100 ml Dimethylformamid mit 6,8 g (40 mmol) Isopropyljodid und 4,2 g Triethylamin 2,5 Stunden auf 70 - 80°C erhitzt. Die Lösung wird im Vakuum eingeengt, der Rückstand mit 60 ml Wasser verrührt und bei pH~ 5 7,7 g 1-Cyclopropyl-6-fluor-1,4-dihydro-7-(4-isopropyl-1-piperazinyl)-4-oxo-3-chinolincarbonsäurehydrojodid vom Schmelzpunkt 288 - 291°C (unter Zersetzung) isoliert.

Zur Überführung in das Betain werden 7,7 g des Hydrojodids in 80 ml Wasser mit einer Lösung von 1,7 g Natriumhydrogencarbonat in 20 ml Wasser versetzt und 3 Stunden bei 20 ° gerührt. Der Niederschlag wird abgesaugt, mit Wasser gewaschen und getrocknet. 4,2 g 1-Cyclopropyl-6-fluor-1,4-dihydro-7-(4-isopropyl-1-piperazinyl)-4-oxo-3-chinolincarbonsäure vom Schmelzpunkt 224 - 227° (unter Zersetzung).

Zur Überführung in das Hydrochlorid werden 10,4 g des Betains in 50 ml Wasser suspendiert, mit 25 ml konzentrierter Salzsäure versetzt und 15 Minuten auf 90° erwärmt. Die Lösung wird filtriert, mit 200 ml Ethanol versetzt und das ausgefallene Salz nach dem Abkühlen abgesaugt, mit Ethanol gewaschen und getrocknet. Man erhält 8,8 g 1-Cyclopropyl-6-fluor-1,4-dihydro-7-(4-isopropyl-1-piperazinyl)-4-oxo-3-chinolincarbonsäure-hydrochlorid vom Schmelzpunkt > 330° (unter Zersetzung).

Beispiel 10

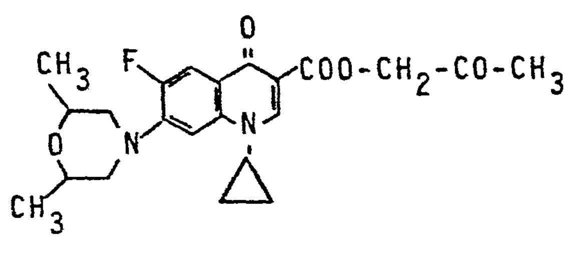

a) 5,6 g (20 mmol) 1-Cyclopropyl-7-chlor-8-fluor-1,4-dihydro-4-oxo-3-chinolincarbonsäure werden in 70 ml Dimethylsulfoxid mit 2,53 g (22 mmol) 2,6-Dimethylmorpholin und 4,4 g 1,4-Diazabicyclo[2,2,2]octan

6 Stunden auf 120°C erhitzt. Das Gemisch wird im Hochvakuum eingeengt, mit 30 ml Wasser verrührt und der Niederschlag abgesaugt, mit Wasser gewaschen und aus Glykolmonomethylether umkristallisiert.
Man erhält 2,4 g (33 % d.Th.) 1-Cyclopropyl-6-fluor-1,4-dihydro-4-oxo-7-(3,5-dimethylmorpholino)-3-chinolincarbonsäure vom Schmelzpunkt 253 - 257°C (unter Zersetzung).

b) 1,8 g (5 mmol) 1-Cyclopropyl-6-fluor-1,4-dihydro-4-oxo-7-(3,5-dimethylmorpholino)-3-chinolincarbonsäure wird mit einer Lösung von 0,2 g (5 mmol) NaOH in 5 ml Wasser in Lösung gebracht, filtriert und das Filtrat zur Trockne eingeengt. Zur restlosen Entfernung von Wasser wird noch dreimal mit jeweils 5 ml Dichlormethan versetzt und eingeengt.
Man erhält 1-Cyclopropyl-6-fluor-1,4-dihydro-4-oxo-7-(3,5-dimethylmorpholino)-3-chinolincarbonsäure-Natriumsalz in praktisch quantitativer Ausbeute.

c) 3,8 g (10 mmol) 1-Cyclopropyl-6-fluor-1,4-dihydro-4-oxo-7-(3,5-dimethylmorpholino)-3-chinolincarbonsäure-Natriumsalz werden in 100 ml absolutem Dimethylformamid suspendiert und mit 2,7 g Chloraceton 24 Stunden bei 25°C gerührt. Man engt im Hochvakuum ein, verrührt den Rückstand mit 100 ml Wasser, saugt den Niederschlag ab, wäscht mit Wasser und Methanol, trocknet im Vakuum und erhält 2,8 g (67 % d.Th.) 1-Cyclopropyl-6-fluor-1,4-dihydro-4-oxo-7-(3,5-dimethylmorpholino)-3-chinolincarbonsäure-(2-oxopropylester) vom Schmelzpunkt 206 - 210°C (unter Zersetzung).

Beispiel 11

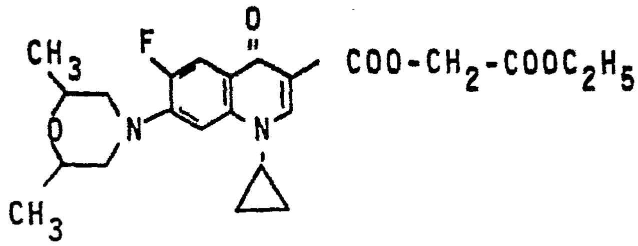

Analog Beispiel 10 c) erhält man mit Bromessigsäureethylester 1-Cyclopropyl-6-fluor-1,4-dihydro-4-oxo-7-(3,5-dimethylmorpholino)-3-chinolincarbonsäure-(ethoxycarbonylmethylester) vom Schmelzpunkt 181 - 183°C.

Beispiel 12

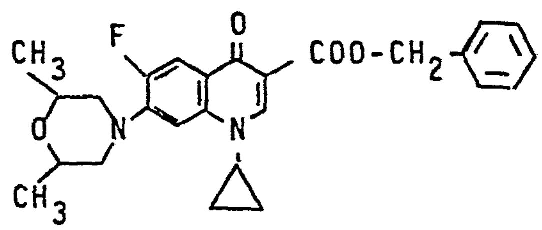

Analog Beispiel 10 c) erhält man mit Benzylchlorid 1-Cyclopropyl-6-fluor-1,4-dihydro-4-oxo-7-(3,5-dimethyl-morpholino)-3-chinolincarbonsäurebenzylester vom Schmelzpunkt 195 - 198°C (unter Zersetzung).

Beispiel 13

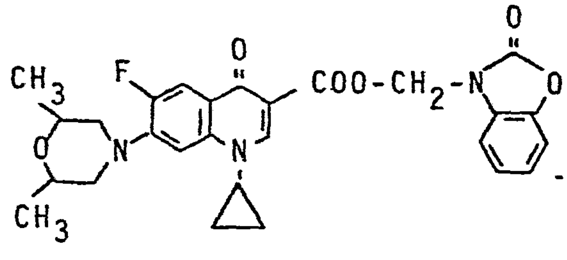

Analog Beispiel 10 c) erhält man mit 3-Chlormethyl-2(3H)-benzoxazolon 1-Cyclopropyl-6-fluor-1,4-dihydro-4-oxo-7-(3,5-dimethylmorpholino)-3-chinolincarbonsäure-/2(3H)-benzoxazolon-3-ylmethylester7 vom Schmelzpunkt 192 bis 195 °C (unter Zersetzung).

## Beispiel 14

Analog Beispiel 1 b) wurden auch die folgenden Verbindungen hergestellt:

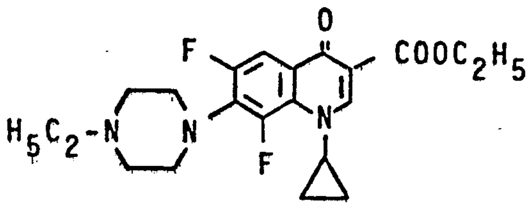

Fp. 182-183°C
(14a)

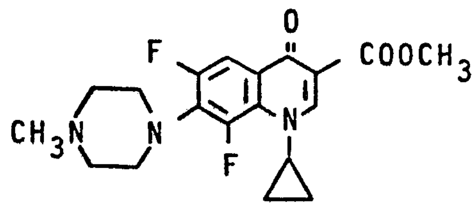

Fp. 218-219°C
(14b)

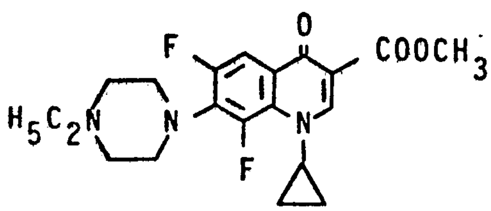

Fp. 203-205°C
(14c)

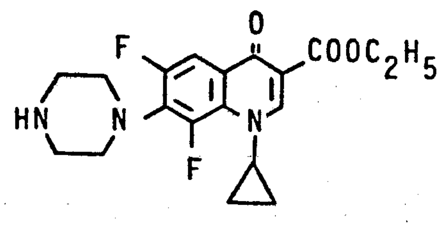

Fp. 202-204°C
(14d)

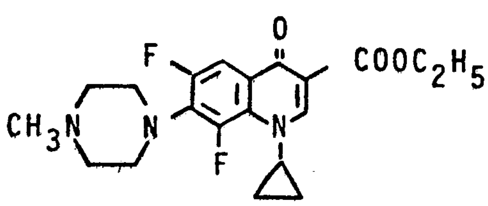

Fp. 175-176°C
(14e)

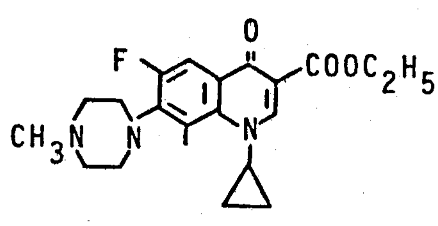

Fp. 202-204°C
(14f)

Die Verbindung 14 e) wurde außerdem auch nach dem Verfahren des Beispiels 1c) hergestellt.

Patentansprüche:

1. Chinoloncarbonsäureester der Formeln (I) oder (II),

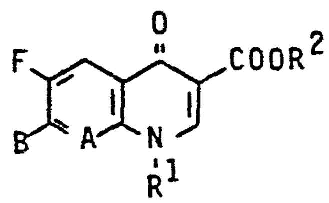

(I)

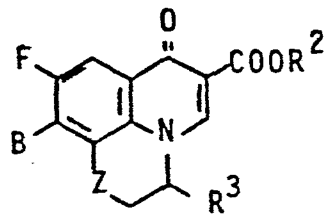

(II)

in denen

A für Stickstoff oder C-$R^4$ steht, wobei $R^4$ Wasserstoff, Fluor, Chlor oder Methyl bedeutet,

B für 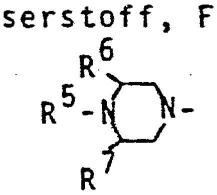 oder 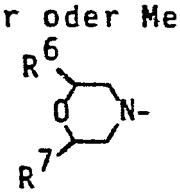 steht und

außerdem B für 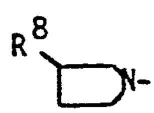 steht, wenn $R^1$ nicht Cyclopropyl bedeutet, worin

$R^5$ Wasserstoff, eine verzweigte oder unverzweigte Alkylgruppe mit 1 bis 4 Kohlenstoffatomen, die gegebenenfalls durch eine Hydroxy- oder Methoxygruppe substituiert sein kann, bedeutet,

$R^6$ für Wasserstoff, Methyl oder Phenyl,

$R^7$ für Wasserstoff oder Methyl und

$R^8$ für Wasserstoff, Hydroxy, Amino, Alkyl- oder Dialkylamino mit 1 oder 2 Kohlenstoffatomen in der Alkylgruppe, Hydroxymethyl, Aminomethyl, Alkyl- oder Dialkylaminomethyl mit 1 oder 2 Kohlenstoffatomen in der Alkylgruppe steht,

$R^1$ einen Alkylrest mit 1 bis 3 Kohlenstoffatomen, Cyclopropyl, 2-Fluorethyl, Methoxy, 4-Fluorphenyl oder Methylamino darstellt,

$R^2$ für gegebenenfalls durch Methoxy oder 2-Methoxyethoxy substituiertes Alkyl mit 1 bis 6 Kohlenstoffatomen sowie Cyclohexyl, Benzyl, 2-Oxopropyl, Phenacyl, Ethoxycarbonylmethyl, Pivaloyloxymethyl steht, sowie

$R^3$ Wasserstoff , Methyl oder Ethyl bedeutet und

Z für Sauerstoff, durch Methyl oder Phenyl substituierten Stickstoff sowie $CH_2$ steht,

und deren pharmazeutisch verwendbare Hydrate und Säureadditionssalze.

2. Chinoloncarbonsäureester der Formel (Ia)

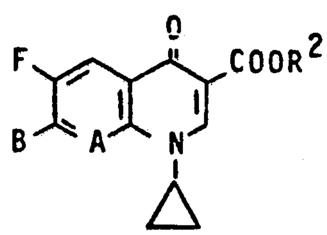

(Ia)

in welcher

B für 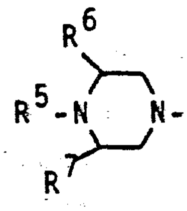 oder 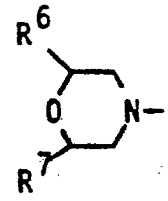 steht,

und

A, $R^2$, $R^5$, $R^6$ und $R^7$ die in Anspruch 1 angegebene Bedeutung haben, sowie deren pharmazeutisch verwendbare Hydrate und Säureadditionssalze.

3. Chinoloncarbonsäureester der Formel (Ia)

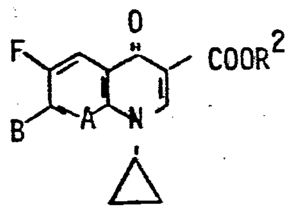 (Ia)

in welcher

$R^2$ für gegebenenfalls durch Methoxy substituiertes Alkyl mit 1 bis 4 Kohlenstoffatomen sowie Benzyl, 2-Oxopropyl, Phenacyl sowie Ethoxycarbonylmethyl steht,

B für 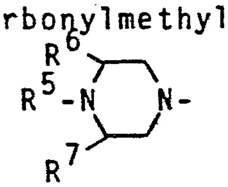 oder 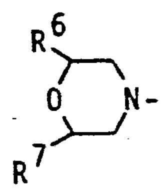 steht,

worin

$R^5$ Wasserstoff, Methyl oder Ethyl bedeutet,

$R^6$ für Wasserstoff oder Methyl,

$R^7$ für Wasserstoff oder Methyl und

A die in Anspruch 1 angegebene Bedeutung hat,

sowie deren pharmazeutisch verwendbare Hydrate und Säureadditionssalze.

4. Verbindungen aus der Gruppe bestehend aus

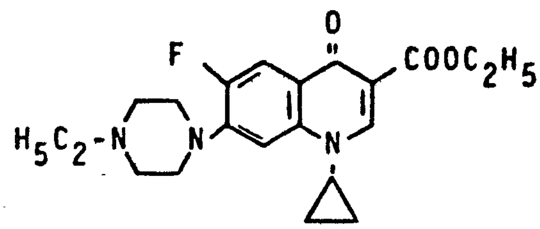

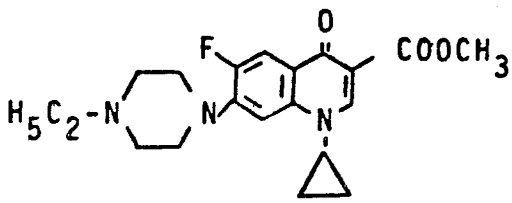

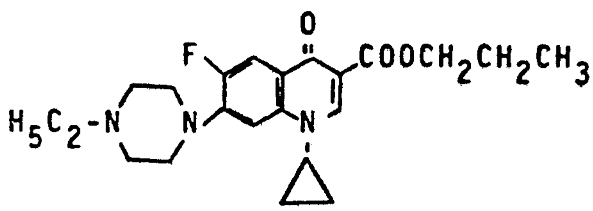

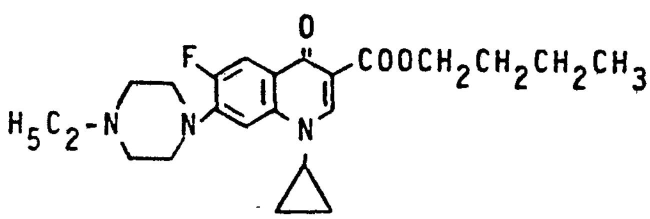

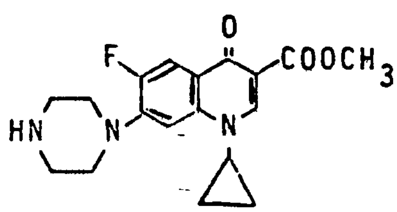

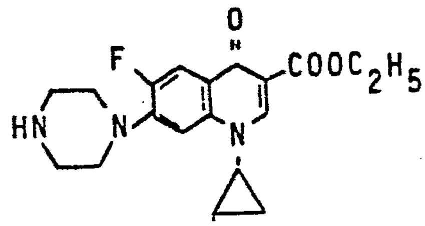

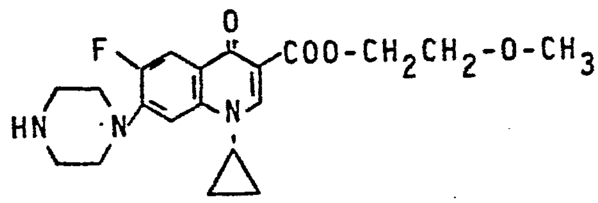

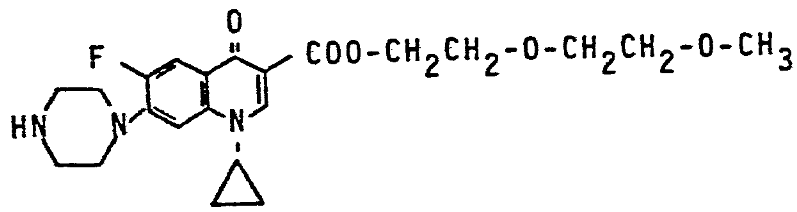

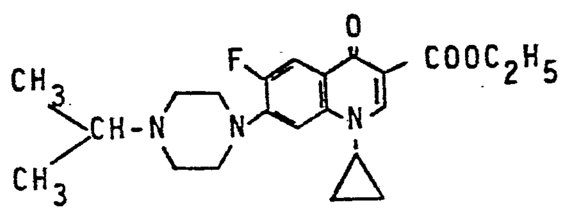

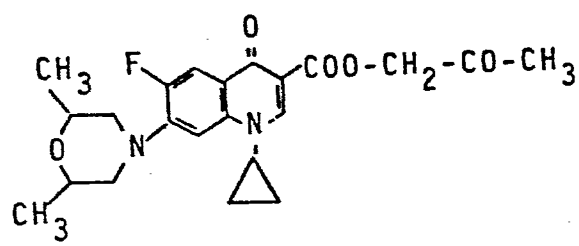

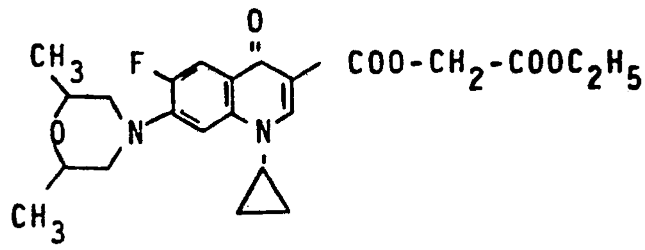

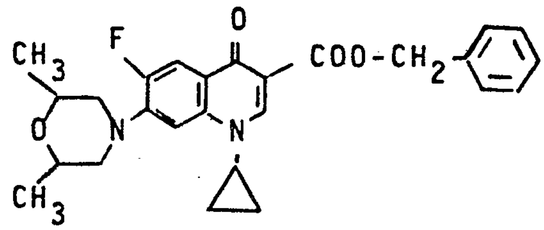

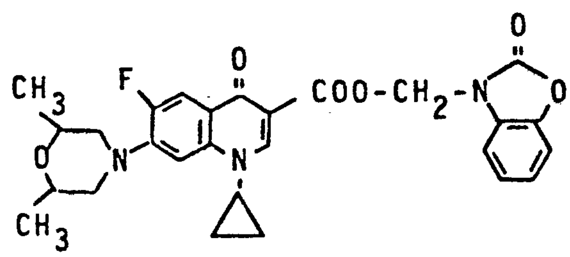

5. Verbindungen aus der Gruppe bestehend aus

1-Ethyl-6-fluor-1,4-dihydro-4-oxo-7-(1-piperazinyl)-chinolin-3-carbonsäureethylester,

1-Ethyl-6-fluor-1,4-dihydro-4-oxo-7-(1-piperazinyl)-chinolin-3-carbonsäuremethylester,

1-Ethyl-6-fluor-1,4-dihydro-4-oxo-7-(1-piperazinyl)-chinolin-3-carbonsäure-n-butylester,

1-Ethyl-6-fluor-1,4-dihydro-7-(4-methyl-1-piperazinyl)-4-oxo-chinolin-3-carbonsäureethylester,

1-Ethyl-6-fluor-1,4-dihydro-4-oxo-7-(1-piperazinyl)-1,8-naphthyridin-3-carbonsäureethylester,

9-Fluor-3-methyl-10-(4-methyl-1-piperazinyl)-7-oxo-2,3-dihydro-7,4-pyrido[1,2,3-de]1,4-benzoxazin-6-carbonsäureethylester,

9-Fluor-5-methyl-8-(4-methyl-1-piperazinyl)-6,7-dihydro-1-oxo-1H,5H-benzo[i,j]chinolicin-2-carbonsäureethylester,

6-Fluor-1-(4-fluorphenyl)-1,4-dihydro-4-oxo-7-(1-piperazinyl)-chinolin-3-carbonsäureethylester,

6-Fluor-1-(2-fluorethyl)-1,4-dihydro-4-oxo-7-(1-piperazinyl)-chinolin-3-carbonsäureethylester,

1-Ethyl-6,8-difluor-1,4-dihydro-4-oxo-7-(1-piperazinyl)-chinolin-3-carbonsäureethylester,

1-Ethyl-6,8-difluor-1,4-dihydro-4-oxo-7-(4-methyl-1-piperazinyl)-chinolin-3-carbonsäure-n-propylester,

1-Cyclopropyl-6,8-difluor-1,4-dihydro-4-oxo-7-(1-piperazinyl)-chinolin-3-carbonsäureethylester,

1-Cyclopropyl-6,8-difluor-1,4-dihydro-4-oxo-7-(4-methyl-1-piperazinyl)-chinolin-3-carbonsäureethylester,

1-Cyclopropyl-6,8-difluor-1,4-dihydro-4-oxo-7-(3-methyl-1-piperazinyl)-chinolin-3-carbonsäure-n-butylester,

1-Cyclopropyl-8-chlor-6-fluor-1,4-dihydro-4-oxo-7-(4-ethyl-1-piperazinyl)-chinolin-3-carbonsäureethylester,

1-Cyclopropyl-6-fluor-1,4-dihydro-4-oxo-8-methyl-7-(4-methyl-1-piperazinyl)-chinolin-3-carbonsäuremethylester und
1-Ethyl-6-fluor-1,4-dihydro-4-oxo-7-(3-amino-1-pyrrolidinyl)-chinolin-3-carbonsäureethylester.

6. Verfahren zur Herstellung von Chinoloncarbonsäureestern der Formeln (I) oder (II)

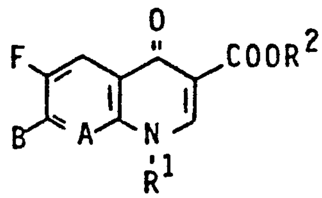 (I)

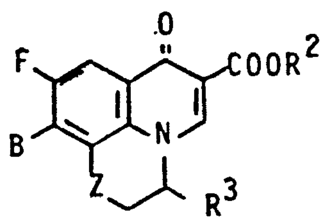 (II)

in denen

A für Stickstoff oder C-$R^4$ steht, wobei

$R^4$ Wasserstoff, Fluor, Chlor oder Methyl bedeutet,

B für 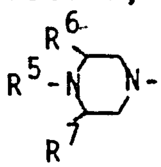 oder 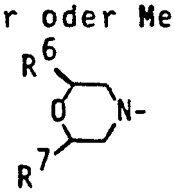 steht und

außerdem B für 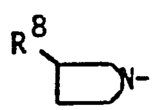 steht, wenn $R^1$ nicht Cyclopropyl bedeutet, worin

$R^5$ Wasserstoff, eine verzweigte oder unverzweigte Alkylgruppe mit 1 bis 4 Kohlenstoffatomen, die gegebenenfalls durch eine Hydroxy- oder Methoxygruppe substituiert sein kann, bedeutet,

$R^6$ für Wasserstoff, Methyl oder Phenyl,

$R^7$ für Wasserstoff oder Methyl und

$R^8$ für Wasserstoff, Hydroxy, Amino, Alkyl- oder Dialkylamino mit 1 oder 2 Kohlenstoffatomen in der Alkylgruppe, Hydroxymethyl, Aminomethyl, Alkyl- oder Dialkylaminomethyl mit 1 oder 2 Kohlenstoffatomen in der Alkylgruppe steht,

$R^1$ einen Alkylrest mit 1 bis 3 Kohlenstoffatomen, Cyclopropyl, 2-Fluorethyl, Methoxy, 4-Fluorphenyl oder Methylamino darstellt,

$R^2$ für gegebenenfalls durch Methoxy oder 2-Methoxyethoxy substituiertes Alkyl mit 1 bis 6 Kohlenstoffatomen sowie Cyclohexyl, Benzyl, 2-Oxopropyl, Phenacyl, Ethoxycarbonylmethyl, Pivaloyloxymethyl steht, sowie

$R^3$ Wasserstoff, Methyl oder Ethyl bedeutet und

Z für Sauerstoff, durch Methyl oder Phenyl substituierten Stickstoff sowie $CH_2$ steht,

und deren pharmazeutisch verwendbaren Hydraten und Säureadditionssalzen, dadurch gekennzeichnet, daß man die entsprechenden Chinoloncarbonsäuren (III) bzw. (IV)

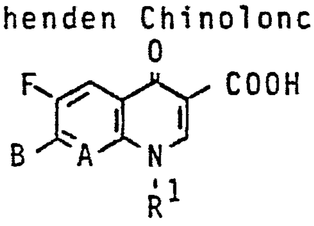

(III)

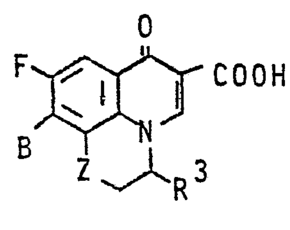

(IV)

in welchen

A, B, $R^1$, $R^3$ und Z die oben angegebene Bedeutung haben, mit Alkoholen der Formel (V)

$$R^2 - OH \qquad (V)$$

in welcher

$R^2$ die oben angegebene Bedeutung hat,

in Gegenwart von starken Säuren umsetzt oder daß man Chinoloncarbonsäureester der Formeln (VI) bzw. (VII)

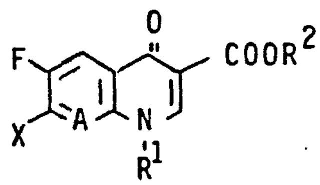

(VI)

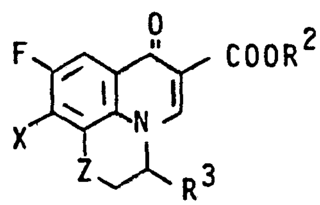

(VII)

in welchen

A, $R^1$, $R^2$, $R^3$ und Z die oben angegebene Bedeutung haben und

X Halogen, bevorzugt Fluor oder Chlor bedeutet,

mit Aminen der Formel (VIII)

$$B - H \qquad (VIII)$$

in welcher

B die oben angegebene Bedeutung hat,

gegebenenfalls in Gegenwart von Säurebindern umsetzt, oder daß man ein Salz einer Carbonsäure der Formeln

(IX) bzw. (X)

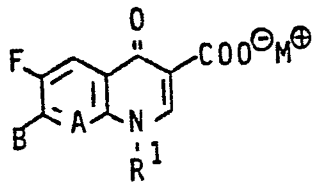

(IX)

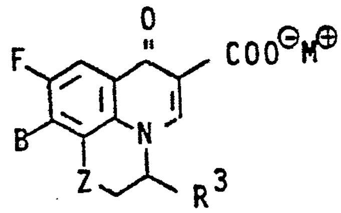

(X)

in welchen

A, B, $R^1$, $R^3$ und Z die oben angegebene Bedeutung haben und

$M^{\oplus}$ für $Na^{\oplus}$, $K^{\oplus}$, 1/2 $Ca^{2\oplus}$ oder $Ag^{\oplus}$ stehen,

mit Verbindungen der Formel (XI)

$$R^2 - Y \qquad (XI)$$

in welcher

$R^2$ die oben angegebene Bedeutung hat und

Y für Halogen, bevorzugt Chlor, Brom oder Jod steht, umsetzt.

7. Parenterales Arzneimittel enthaltend Chinoloncarbonsäureester der Formeln (I) oder (II)

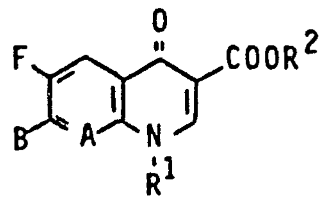

(I)

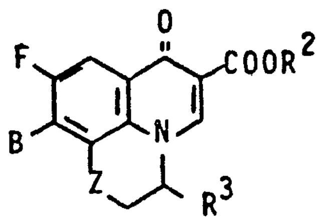

(II)

in denen

A für Stickstoff oder C-$R^4$ steht, wobei

$R^4$ Wasserstoff, Fluor, Chlor oder Methyl bedeutet,

B für 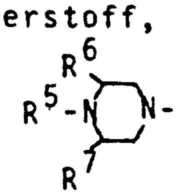 oder 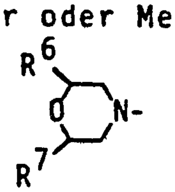 steht und

außerdem B für 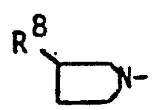 steht, wenn $R^1$ nicht Cyclopropyl bedeutet, worin

$R^5$ Wasserstoff, eine verzweigte oder unverzweigte Alkylgruppe mit 1 bis 4 Kohlenstoffatomen, die gegebenenfalls durch eine Hydroxy- oder Methoxygruppe substituiert sein kann, bedeutet,

$R^6$ für Wasserstoff, Methyl oder Phenyl,

$R^7$ für Wasserstoff oder Methyl und

$R^8$ für Wasserstoff, Hydroxy, Amino, Alkyl- oder Dialkylamino mit 1 oder 2 Kohlenstoffatomen in der Alkylgruppe, Hydroxymethyl, Aminomethyl, Alkyl- oder Dialkylaminomethyl mit 1 oder 2 Kohlenstoffatomen in der Alkylgruppe steht,

$R^1$ einen Alkylrest mit 1 bis 3 Kohlenstoffatomen, Cyclopropyl, 2-Fluorethyl, Methoxy, 4-Fluorphenyl oder Methylamino darstellt,

$R^2$ für gegebenenfalls durch Methoxy oder 2-Methoxyethoxy substituiertes Alkyl mit 1 bis 6 Kohlenstoffatomen sowie Cyclohexyl, Benzyl, 2-Oxopropyl, Phenacyl, Ethoxycarbonylmethyl, Pivaloyloxymethyl steht, sowie

$R^3$ Wasserstoff, Methyl oder Ethyl bedeutet und

Z für Sauerstoff, durch Methyl oder Phenyl substituierten Stickstoff sowie $CH_2$ steht,

und deren pharmazeutisch verwendbare Hydrate und Säureadditionssalze.

8. Verwendung von Chinoloncarbonsäureestern der Formeln (I) oder (II)

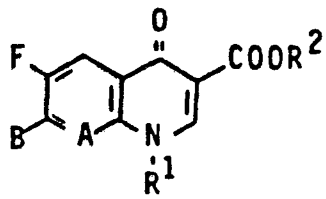

(I)

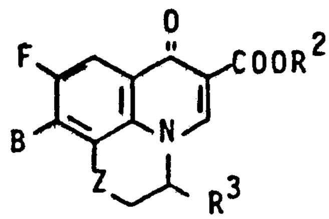

(II)

in denen

A für Stickstoff oder C-$R^4$ steht, wobei

$R^4$ Wasserstoff, Fluor, Chlor oder Methyl bedeutet,

B für 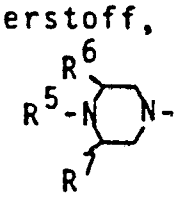 oder 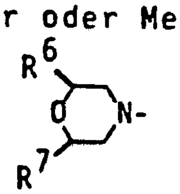 steht und

außerdem B für 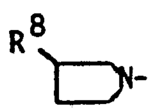 steht, wenn $R^1$ nicht Cyclopropyl bedeutet, worin

$R^5$ Wasserstoff, eine verzweigte oder unverzweigte Alkylgruppe mit 1 bis 4 Kohlenstoffatomen, die gegebenenfalls durch eine Hydroxy- oder Methoxygruppe substituiert sein kann, bedeutet,

$R^6$ für Wasserstoff, Methyl oder Phenyl,

$R^7$ für Wasserstoff oder Methyl und

$R^8$ für Wasserstoff, Hydroxy, Amino, Alkyl- oder Dialkylamino mit 1 oder 2 Kohlenstoffatomen in der Alkylgruppe, Hydroxymethyl, Aminomethyl, Alkyl- oder Dialkylaminomethyl mit 1 oder 2 Kohlenstoffatomen in der Alkylgruppe steht,

$R^1$ einen Alkylrest mit 1 bis 3 Kohlenstoffatomen, Cyclopropyl, 2-Fluorethyl, Methoxy, 4-Fluorphenyl oder Methylamino darstellt,

$R^2$ für gegebenenfalls durch Methoxy oder 2-Methoxyethoxy substituiertes Alkyl mit 1 bis 6 Kohlenstoffatomen sowie Cyclohexyl, Benzyl, 2-Oxopropyl, Phenacyl, Ethoxycarbonylmethyl, Pivaloyloxymethyl steht, sowie

$R^3$ Wasserstoff, Methyl oder Ethyl bedeutet und

Z für Sauerstoff, durch Methyl oder Phenyl substituierten Stickstoff sowie $CH_2$ steht,

und deren pharmazeutisch verwendbaren Hydraten und Säureadditionssalzen zur Herstellung von insbesondere parenteralen Arzneimitteln.

9. Verwendung von Chinoloncarbonsäureestern der Formeln (I) oder (II)

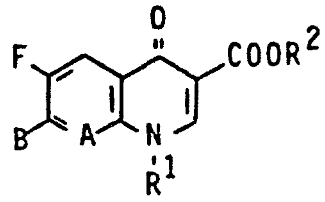

(I)

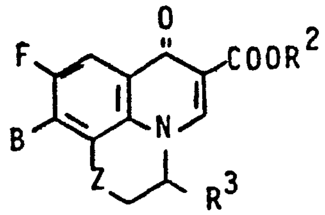

(II)

in denen

A für Stickstoff oder C-$R^4$ steht, wobei

$R^4$ Wasserstoff, Fluor, Chlor oder Methyl bedeutet,

B für 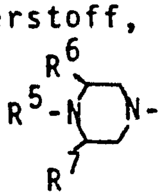 oder 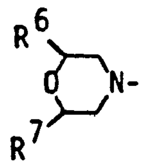 steht und

außerdem B für 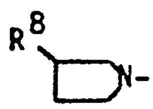 steht, wenn $R^1$ nicht Cyclopropyl bedeutet , worin

$R^5$ Wasserstoff, eine verzweigte oder unverzweigte Alkylgruppe mit 1 bis 4 Kohlenstoffatomen, die gegebenenfalls durch eine Hydroxy- oder Methoxygruppe substituiert sein kann, bedeutet,

$R^6$ für Wasserstoff, Methyl oder Phenyl,

$R^7$ für Wasserstoff oder Methyl und

$R^8$ für Wasserstoff, Hydroxy, Amino, Alkyl- oder Dialkylamino mit 1 oder 2 Kohlenstoffatomen in der Alkylgruppe, Hydroxymethyl, Aminomethyl, Alkyl- oder Dialkylaminomethyl mit 1 oder 2 Kohlenstoffatomen in der Alkylgruppe steht,

$R^1$ einen Alkylrest mit 1 bis 3 Kohlenstoffatomen, Cyclopropyl, 2-Fluorethyl, Methoxy, 4-Fluorphenyl oder Methylamino darstellt,

$R^2$ für gegebenenfalls durch Methoxy oder 2-Methoxyethoxy substituiertes Alkyl mit 1 bis 6 Kohlenstoffatomen sowie Cyclohexyl, Benzyl, 2-Oxopropyl, Phenacyl, Ethoxycarbonylmethyl, Pivaloyloxymethyl steht, sowie

$R^3$ Wasserstoff, Methyl oder Ethyl bedeutet und

Z für Sauerstoff, durch Methyl oder Phenyl substituierten Stickstoff sowie $CH_2$ steht,

und deren pharmazeutisch verwendbaren Hydraten und Säureadditionssalzen bei der insbesondere parenteralen Bekämpfung von Krankheiten des menschlichen oder tierischen Körpers.

10. Verwendung von Chinoloncarbonsäureestern der Formeln (I) oder (II)

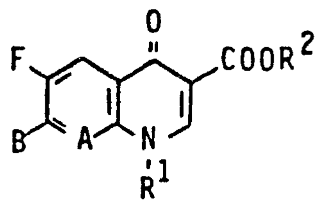

(I)

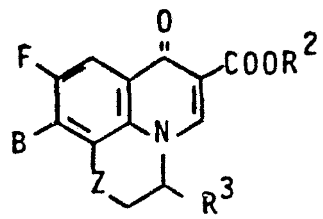

(II)

in denen

A für Stickstoff oder C-$R^4$ steht, wobei

$R^4$ Wasserstoff, Fluor, Chlor oder Methyl bedeutet,

B für 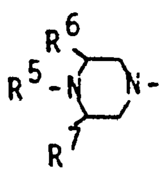 oder 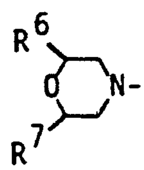 steht und

außerdem B für 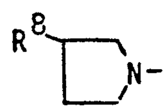 steht, wenn $R^1$ nicht Cyclopropyl bedeutet, worin

$R^5$ Wasserstoff, eine verzweigte oder unverzweigte Alkylgruppe mit 1 bis 4 Kohlenstoffatomen, die gegebenenfalls durch eine Hydroxy- oder Methoxygruppe substituiert sein kann, bedeutet,

$R^6$ für Wasserstoff, Methyl oder Phenyl,

$R^7$ für Wasserstoff oder Methyl und

$R^8$ für Wasserstoff, Hydroxy, Amino, Alkyl- oder Dialkylamino mit 1 oder 2 Kohlenstoffatomen in der Alkylgruppe, Hydroxymethyl, Aminomethyl,

Alkyl- oder Dialkylaminomethyl mit 1 oder 2 Kohlenstoffatomen in der Alkylgruppe steht,

$R^1$ einen Alkylrest mit 1 bis 3 Kohlenstoffatomen, Cyclopropyl, 2-Fluorethyl, Methoxy, 4-Fluorphenyl oder Methylamino darstellt,

$R^2$ für gegebenenfalls durch Methoxy oder 2-Methoxyethoxy substituiertes Alkyl mit 1 bis 6 Kohlenstoffatomen sowie Cyclohexyl, Benzyl, 2-Oxopropyl, Phenacyl, Ethoxycarbonylmethyl, Pivaloyloxymethyl steht, sowie

$R^3$ Wasserstoff, Methyl oder Ethyl bedeutet und

Z für Sauerstoff, durch Methyl oder Phenyl substituierten Stickstoff sowie $CH_2$ steht,

und deren pharmazeutisch verwendbaren Hydraten und Säureadditionssalzen zur qualitativen und/oder quantitativen Verbesserung tierischer Erzeugnisse.

11. 1-Cyclopropyl-6-fluor-1,4-dihydro-7-(4-isopropyl-1-piperazinyl)-4-oxo-3-chinolincarbonsäure sowie deren pharmazeutisch verwendbare Hydrate und Säureadditionssalze.

12. Verfahren zur Herstellung von 1-Cyclopropyl-6-fluor-1,4-dihydro-7-(4-isopropyl-1-piperazinyl)-4-oxo-3-chinolincarbonsäure, dadurch gekennzeichnet, daß man 1-Cyclopropyl-6-fluor-1,4-dihydro-4-oxo-7-(1-piperazinyl)-3-chinolincarbonsäure in einem Lösungsmittel mit Isopropylhalogenid in Gegenwart eines Amins umsetzt und gegebenenfalls die freie Säure aus dem anfallenden Hydrohalogenid isoliert.

13. Verwendung von 1-Cyclopropyl-6-fluor-1,4-dihydro-7-(4-isopropyl-1-piperazinyl)-4-oxo-3-chinolincarbonsäure zur Herstellung von Arzneimitteln.